# EUROPÄISCHE PATENTSCHRIFT

(11) **EP 2 368 596 B1**
(45) Veröffentlichungstag und Bekanntmachung des Hinweises auf die Patenterteilung: **08.05.2013**
(21) Anmeldenummer: 11158803.4
(22) Anmeldetag: 18.03.2011
(51) Int. Cl.: A61M 25/02, A61M 25/06, A61F 13/02, A61M 5/32, A61M 5/158

(54) **Kanülenanordnung**
Cannula assembly
Agencement de canule

(30) Priorität: 23.03.2010 DE 202010000443 U
(43) Veröffentlichungstag der Anmeldung: 28.09.2011
(73) Patentinhaber: sfm medical devices GmbH, 63607 Wächtersbach (DE)
(72) Erfinder: Kehr, Markus, 63599, Biebergemünd (DE)
(74) Vertreter: Stoffregen, Hans-Herbert

(56) Entgegenhaltungen:
- EP-A1- 2 119 462
- DE-A1- 10 059 519
- US-A1- 2004 260 250
- US-B1- 6 436 073

## Beschreibung

Die Erfindung bezieht sich auf eine Kanülenanordnung umfassend eine Kanüle mit einem proximalen und einem distalen Abschnitt, die vorzugsweise zueinander einen rechten oder im Wesentlichen einen rechten Winkel einschließen, eine von dem proximalen Abschnitt ausgehende Handhabe mit zumindest einem flächigen ersten Abschnitt, der von einem von dem proximalen Abschnitt der Kanüle ausgehenden Mittelabschnitt ausgeht oder Abschnitt von diesem ist, sowie eine Fixierungshilfe mit einer Klebefläche zum Fixieren der Kanülenanordnung auf einer Unterlage wie Haut eines Patienten.

Abgewinkelte Kanülen mit flügelartiger Handhabe sind als Portkanülen bekannt, die dazu bestimmt sind, einen in einem Körper implantierten Port mit Medikamenten zu versorgen. Hierzu weist der Port eine Membran mit einer Dicke z. B. zwischen 3 mm und 5 mm und einem Durchmesser von beispielhaft 10 mm bis 20 mm auf, die von dem distalen Abschnitt der Kanüle durchstochen werden muss, um sodann das Medikament zuzuführen. Dabei wird der proximale Abschnitt mittels der Flügel auf der Haut des Patienten fixiert. Der proximale Abschnitt ist mit einem abklemmbaren Schlauch verbunden, der endseitig z. B. einen Luer-Adapter mit Drehverschluss aufweisen kann, um eine Verbindung zu einem Medikamentenbehältnis zu ermöglichen.

Um nach Gebrauch der Kanüle, also nach dem Entfernen aus dem Körper die die Kanüle handhabende Person zu schützen, wird der distale Abschnitt von einer Schutzeinrichtung abgedeckt, die nach der US-B-6,500,155 durch die Flügel der Handhabe selbst gebildet wird.

Der JP-A-2007195827 ist eine Portkanüle zu entnehmen, bei der ein flexibles Rohr über den distalen Abschnitt zu dessen Abdeckung geschoben werden kann.

Eine Portkanüle nach der DE-T-699 35 347 weist gleichfalls eine Flügelanordnung als Handhabe auf.

Die US-A-2006/0030825 sieht eine Schutzeinrichtung für einen distalen Abschnitt einer Portkanüle vor, die als gesondertes Teil ausgebildet ist und nach Gebrauch der Kanüle diese rastend umgibt.

In der US-A-6,921,388 wird eine Portkanüle mit einer Schutzkappe beschrieben, die verschwenkbar ist, um einen Schutz nach deren Gebrauch zu ermöglichen.

Die WO-A-2002/45574 bezieht sich auf eine Portkanüle mit Schutzkappe, die in Längsrichtung der Kanüle verschiebbar ist.

Eine weitere Portkanüle ist der WO-A-2005/00377 zu entnehmen, die verschwenkbare Flügel als Handhabe aufweist, die von einem Mittelabschnitt ausgehen, der proximalen Abschnitt der Kanüle umgibt.

Bei einer Portkanüle nach der DE-A-10 2008 002 854 (= EP-A-2 119 462) kann die Kanüle nach deren Gebrauch von einer verschwenkbaren Schutzeinrichtung abgedeckt werden, die nach Umschließen der Kanüle nicht mehr entfernbar ist.

Entsprechende Kanülen werden während der Behandlung, also dann, wenn der distale Abschnitt in einem Port eingesteckt ist, üblicherweise mittels eines Pflasters auf der Haut des Patienten befestigt. Zum Entfernen der Kanüle wird zunächst das Pflaster entfernt. Hierbei erfolgt häufig ein Verrutschen der Kanüle, wodurch ggf. der Port beschädigt werden kann. Außerdem werden dem Patienten Schmerzen zugefügt.

Sowohl der US-A-2007/0149920 als auch der US-A-4,645,495 sind Portkanülen zu entnehmen, die mit einem kreisscheibenförmigen Element auf der Haut eines Patienten auflegbar und fixierbar sind. Hierzu sieht die US-A-4,645,495 eine Klebefläche auf der Unterseite des kreisscheibenförmigen Elementes vor, die ein Haften auf der Haut ermöglicht.

Eine Portkanüle nach der US-A-2004/0260250 kann über ein Pflaster auf einer Haut fixiert werden, das sich über der Handhabe der Kanüle erstreckt.

Zum Fixieren von insbesondere Infusionskanülen auf der Haut eines Patienten sieht die DE-A-100 59 519 einen Klebebandstreifen vor, der sich über Haltelaschen der Kanüle erstreckt. Eine entsprechende Anordnung ist der US-B-6 436 073 zu entnehmen.

Nach Ablegen einer Kanüle wird nach der US-A-2007/0112307 ein Klebeband benutzt, das sich über flügelartige Handhaben erstreckt.

Um einen Katheter nach der US-A-4,250,880 auf der Haut eines Patienten zu fixieren, weisen als Handhabe dienende Flügel unterseitig ein Klebemittel auf, um auf einer Haut fixiert zu werden. Zusätzlich können Pflaster benutzt werden, die sich über der Handhabe erstrecken.

Um eine Kanüle an einem Arm zu fixieren, ist nach der US-A-5,024,665 ein den Arm umgebendes Band mit einer Handhabe der Kanüle verbunden, wobei das Band mittels eines Klettverschlusses gesichert ist.

Der vorliegenden Erfindung liegt die Aufgabe zu Grunde, eine Kanülenanordnung der eingangs genannten Art, insbesondere eine Portkanüle so weiterzubilden, dass ein problemloses Fixieren der Kanüle bei der Benutzung erfolgen kann, gleichzeitig jedoch sichergestellt chergestellt ist, dass beim Entfernen der Fixierung die Kanüle nicht unkontrolliert verschoben werden kann, so dass Beschädigungen des Ports oder das Verursachen von Schmerzen vermieden werden.

Zur Lösung der Aufgabe sieht die Erfindung vor, dass sich entlang des flächigen Abschnitts der Handhabe und seitlich über diesen überstehend die Fixierungshilfe mit der Klebefläche erstreckt, dass die Klebefläche der Fixierungshilfe auf der dem flächigen Abschnitt zugewandten Seite verläuft und dass die Fixierungshilfe in dem den Mittelabschnitt abdeckenden Bereich eine Klebefläche nicht aufweist.

Abweichend vom vorbekannten Stand der Technik wird die Handhabe über eine Fixierungshilfe wie Pflaster fixiert, die bzw. das im Bereich der Handhabe eine Klebefläche nicht aufweist, so dass ein Lösen von der Haut des Patienten erfolgt, ohne dass die Handhabe selbst mitgenommen wird. Somit ist die Gefahr von Beschädigungen des Portes oder das Auslösen von Schmerzen ausgeschlossen.

Die Fixierungshilfe - nachstehend vereinfacht, jedoch nicht schutzeinschränkend, als Pflaster bezeichnet - selbst ist lösbar und damit problemlos entfernbar mit der Handhabe bzw. dem Mittelabschnitt verbunden.

Damit das Pflaster eindeutig zu der Handhabe fixiert ist und somit ein problemloses Befestigen der Kanüle über das Pflaster möglich ist, ohne dass die Klebefläche benötigt wird, wird das Pflaster insbesondere mit mechanischen Hilfsmitteln auf dem Mittelabschnitt fixiert. Hierzu ist vorzugsweise vorgesehen, dass das Pflaster zwischen dem Mittelabschnitt und einer Schutzeinrichtung fixiert ist, die ihrerseits auf dem Mittelabschnitt verrastet. Die Schutzeinrichtung ist dafür bestimmt, dass nach Benutzung der Kanüle der distale Abschnitt abgedeckt wird.

Hierzu wird bei der Anlieferung der Kanüle die Schutzeinrichtung auf dem Mittelabschnitt verrastet, so dass infolgedessen eine eindeutige Fixierung und Positionierung des Pflasters zwischen dem Mittelabschnitt der Handhabe und der Schutzeinrichtung sichergestellt ist.

Während der Anlieferung ist der distale Abschnitt üblicherweise von einem Schlauchabschnitt abgedeckt.

Das Pflaster selbst weist vorzugsweise jeweils einen entlang jeden Flügels sich erstreckenden Abschnitt trapezförmiger Geometrie auf, wobei die kürzere Basis im Bereich des Mittelabschnitts verläuft. Die äußeren Ecken sollten abgerundet werden.

Die Fläche des Pflasters (Fixierungshilfe) sollte in etwa 4 - 6 mal größer als die der Flügel der Handhabe sein. Beispielhaft können die Flügel eine Fläche von 5 cm² - 8 cm² und die Fixierungshilfe eine Fläche von 20 cm² - 48 cm², insbesondere von in etwa 30 cm² aufweisen.

Um die Schutzeinrichtung eindeutig auf dem Mittelabschnitt zu fixieren, weist die Schutzeinrichtung in ihrer Bodenwandung ein erstes Rastmittel auf, dem ein in dem Mittelabschnitt der Handhabe vorhandenes zweites Rastmittel zugeordnet ist. Dabei kann das erste Rastmittel eine Aussparung und das zweite Rastmittel ein Vorsprung oder umgekehrt sein.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung von der Zeichnung zu entnehmenden bevorzugten Ausführungsbeispielen.

Es zeigen:
- Fig. 1: eine erfindungsgemäße Kanülenanordnung in perspektivischer Darstellung, von oben betrachtet,
- Fig. 2: die Kanülenanordnung gemäß Fig. 1 in Seitenansicht,
- Fig. 3: die Kanülenanordnung gemäß Fig. 1 und 2 in perspektivischer Darstellung, von unten betrachtet, und
- Fig. 4: die Kanülenanordnung gemäß Fig. 1 - 3 in perspektivischer Darstellung, von oben betrachtet.

Die Erfindung wird nachstehend anhand einer Portkanüle näher erläutert, ohne dass hierdurch eine Beschränkung der erfindungsgemäßen Lehre erfolgen soll. Vielmehr gilt diese für alle Kanülenanordnungen, die über eine Fixierungshilfe wie Pflaster oder ein gleich wirkendes Mittel befestigt werden, um die Fixierungshilfe wie das Pflaster oder das gleich wirkende Mittel unabhängig von der Kanülenanordnung entfernen zu können.

Nachstehend wird die Fixierungshilfe vereinfacht als Pflaster bezeichnet, ohne dass hierdurch die Erfindung eingeschränkt werden soll.

In den Figuren, in denen gleiche Elemente mit gleichen Bezugszeichen versehen sind, ist eine Kanülenanordnung 10 dargestellt, die eine Portkanüle 12 mit Handhabe 14 sowie Schutzeinrichtung 16 umfasst, wie diese der EP-A-2 119 462 (DE-A-10 2008 002 854) zu entnehmen ist, auf deren Offenbarung ausdrücklich Bezug genommen wird und deren Inhalt zur vorliegenden Offenbarung gehört.

Die Kanüle 12 umfasst einen proximalen Abschnitt 18 und einen zu diesem senkrecht verlaufenden distalen Abschnitt 20, der distal eine geschliffene Spitze 23 aufweist, die in einen in einem Körper eines Patienten implantierten Port einstechbar ist, um ein Medikament zuzuführen.

Auf das Ende des proximalen Abschnitts ist in gewohnter Weise ein Schlauch 22 aufschiebbar, der über eine nicht dargestellte Schlauchklemme absperrbar bzw. freigebbar ist. Ferner kann in gewohnter Weise am Ende des Schlauchs ein Luer-Adapter mit Drehverschluss vorgesehen sein.

Die Handhabe 14 weist einen den proximalen Abschnitt der Portkanüle 12 umgebenden zylindrischen oder quaderförmigen Mittelabschnitt 30 auf, von dem Flügel 36, 38 ausgehen, die zu dem Mittelabschnitt 30 verschwenkbar sein sollten. Unterseitig sind die Flügel 36, 38 strukturiert, um beim Positionieren der Kanüle 12 auf einer Patientenhaut eine flächige Auflage zu vermeiden, gleichzeitig jedoch eine sichere Fixierung zu ermöglichen.

Des Weiteren ist im Ausführungsbeispiel - jedoch nicht schutzeinschränkend - integral mit der Handhabe 14 die Schutzeinrichtung 16 ausgebildet wie gespritzt, wobei zwischen dem Mittelabschnitt 30 und der Schutzeinrichtung 16 zum Verschwenken dieser ein Filmscharnier 40 verläuft, durch das eine senkrecht zu der Kanüle 12, also sowohl zu dem proximalen Abschnitt als auch zu dem distalen Abschnitt 20 sich erstreckende Schwenkachse vorgegeben wird. Ferner verläuft das Filmscharnier 40 und somit die Schwenkachse parallel zu einer Ebene, die durch die Flügel 36, 38 in unverschwenktem Zustand aufgespannt ist, wie dies den Figuren zu entnehmen ist. Das Filmscharnier 40 gibt folglich die Schwenkachse vor, um einen kanalförmigen Abschnitt der Schutzeinrichtung 16 zu der Handhabe hin bzw. weg von dieser verschwenken zu können.

Die Schutzeinrichtung 16 weist Seitenwandungen 42, 44 auf, die über eine Bodenwandung 46 miteinander verbunden sind.

Von jeder Innenfläche der Seitenwandungen 42, 44 gehen mehrere Vorsprünge 45 aus, die in einem Abstand zueinander und zu der Innenfläche der Bodenwandung 46 derart verlaufen, dass dann, wenn der distale Abschnitt 20 der Kanüle 12 von der Schutzeinrichtung 16 aufgenommen ist, diese zwischen den Vorsprüngen 45 und der Bodenwandung 46 gesichert ist.

Die Bodenwandung 46 geht in eine flächige Erweiterung 52 über, die über das Filmscharnier 40 mit dem mittleren Abschnitt 30 der Handhabe 14 verbunden ist.

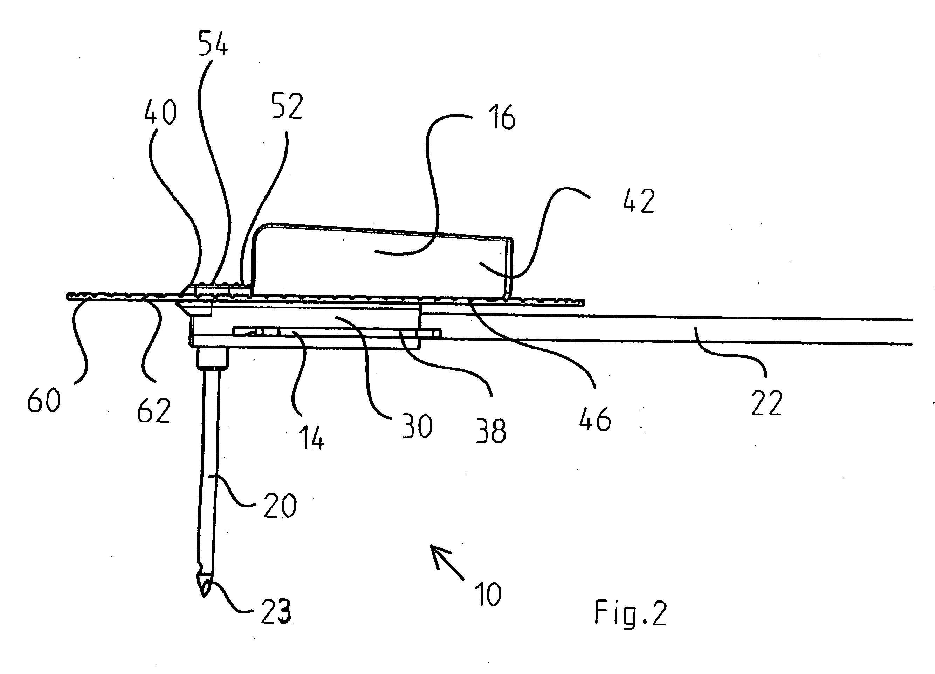

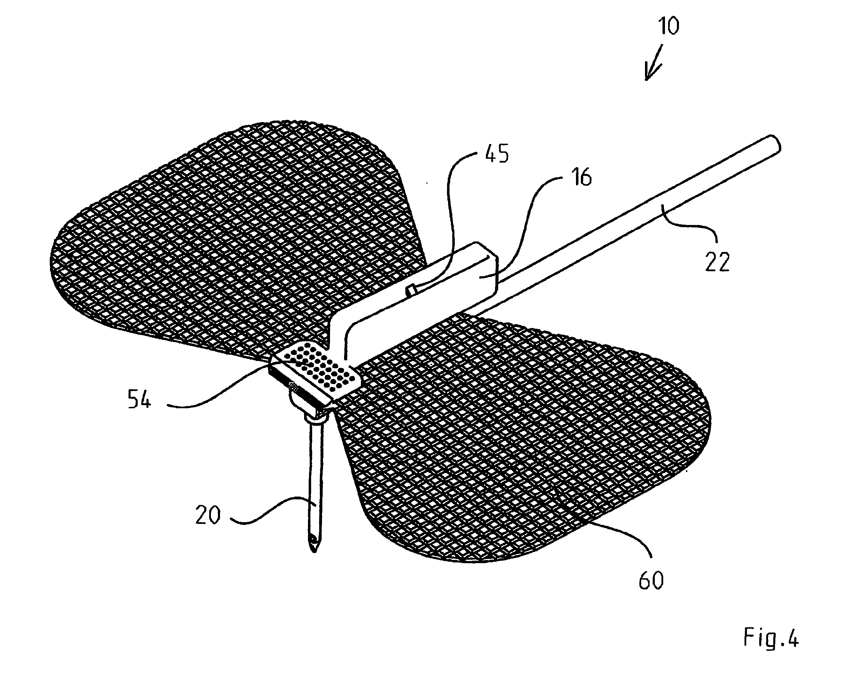

Die flächige Erweiterung 52 ist auf ihrer bei Nutzung der Kanüle 10 (Fig. 2, Fig. 4) freien äußeren Fläche strukturiert (dargestellt sind Vorsprünge 54), um beim Setzen der Kanüle 12, d. h. Einstechen des distalen Abschnitts 20, als Positionierhilfe zu dienen. So steht dem Nutzer beim Setzen der Kanüle, bei der die Flügel 36, 38 aufeinander liegen, um ein Hantieren zu ermöglichen, eine relativ große Fläche zur Verfügung, um den distalen Abschnitt 20 der Kanüle 12 führen zu können, damit ein sicheres Einstechen der Spitze 22 in einen Port ermöglicht wird. Dabei erstreckt sich die Erweiterung 52 in Bezug auf den distalen Abschnitt 20 derart, dass die Längsachse des distalen Abschnitts 20 die Erweiterung 52 vorzugsweise mittig durchsetzt.

Sowohl im Anlieferungs- als auch im Nutzungszustand ist die Schutzeinrichtung 16 auf den Mittelabschnitt 30 fixiert. Hierzu ragt von dem Mittelabschnitt 30 ein Vorsprung 56 ab, dem eine Öffnung 58 in der Bodenwandung 46 zugeordnet ist, wobei der endseitig knopfartig ausgebildete Vorsprung 56 die Öffnung 58 durchsetzt, so dass ein sicheres Fixieren der Schutzeinrichtung 16 sichergestellt ist.

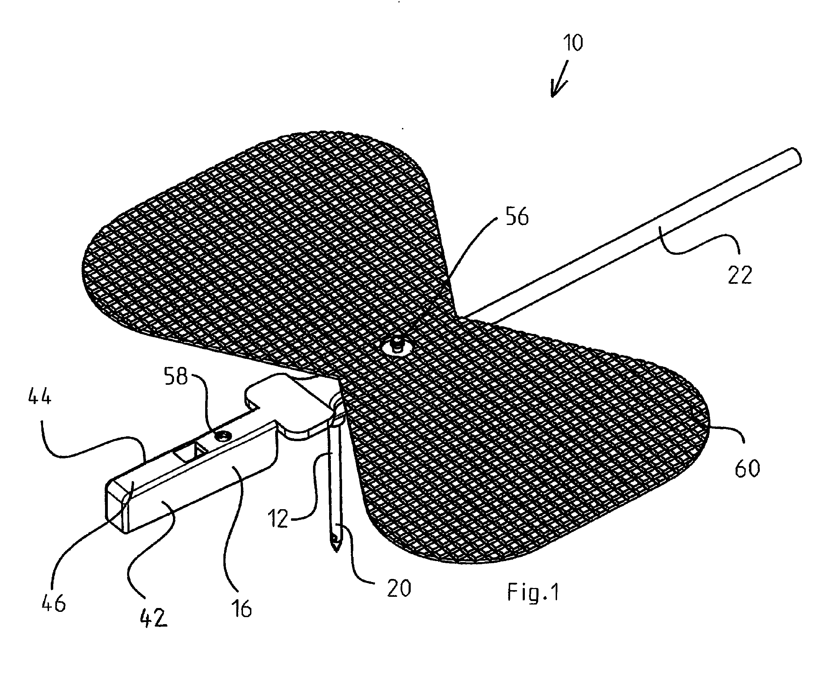

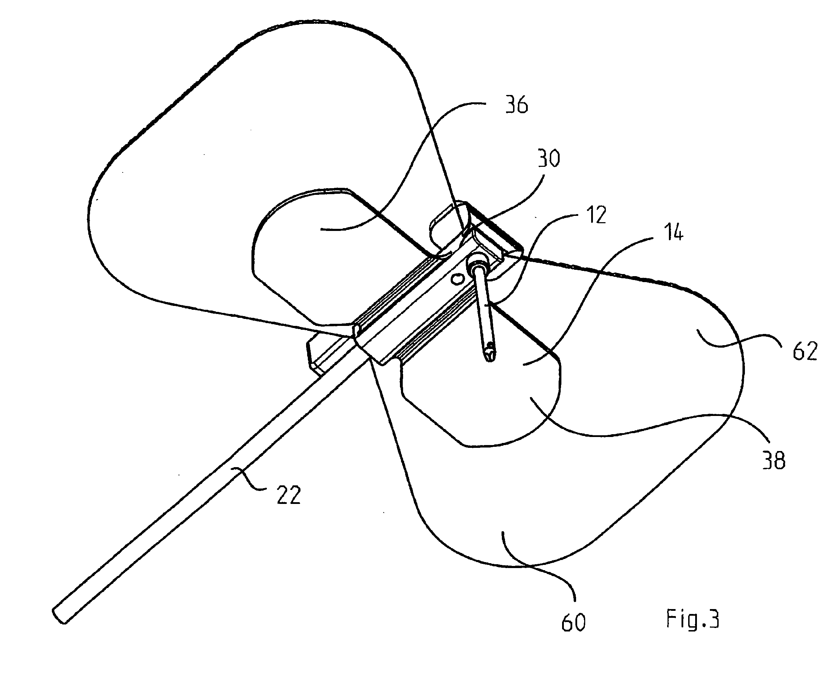

Erfindungsgemäß erstreckt sich zwischen der Schutzeinrichtung 16 und dem Mittelabschnitt 30 ein Pflaster 60, das zwischen der Schutzeinrichtung 16 und dem Mittelabschnitt 30 fixiert wird. Das Pflaster 60 dient dazu, um die Kanülenanordnung 10 auf der Haut eines Patienten zu fixieren, wenn der distale Abschnitt 20 der Kanüle 12 in einen Port eingestochen ist. Das Pflaster 60 erstreckt sich - wie insbesondere Fig. 3 verdeutlicht - seitlich über die Flügel 36, 38 der Handhabe und bietet somit eine hinreichend große Klebefläche 62, auf der der Patientenhaut zugewandten Seite. Allerdings ist erfindungsgemäß vorgesehen, dass das Pflaster 60 im Bereich der Handhabe 14, also der Flügel 36, 38 und des Mittelabschnitts 30, eine Klebefläche nicht aufweist, so dass beim Lösen des Pflasters 60 die Kanülenanordnung 10 nicht mitgenommen wird. Zum Lösen des Pflasters 60 wird die Schutzeinrichtung 16 verschwenkt (Fig. 1), so dass sodann problemlos das Pflaster 60 von der Haut abgezogen werden kann. Hierbei erfolgt ein Verrutschen der Kanüle 10 bzw. deren distalen Abschnitts 12 nicht, da eine klebende Verbindung zwischen dem Pflaster 60 und den Elementen der Kanülenanordnung 10 aufgrund der Aussparung des Klebebereichs nicht erfolgt. Somit kann beim Entfernen des Pflasters 60 ein unkontrolliertes Verschieben der Kanülenanordnung 10 nicht erfolgen, so dass weder der Port beschädigt noch dem Patienten Schmerzen zugefügt werden.

Die Fläche des Pflasters 60 sollte in etwa 4 - 6-fach größer als die der Flügel 36, 38 sein, die eine Fläche von 6 cm² - 7 cm² aufweisen können. Die bevorzugte flächige Erstreckung des Pflasters 60 liegt zwischen 25 cm² und 35 cm², insbesondere in etwa bei 30 cm².

## Patentansprüche

1. Kanülenanordnung (10) umfassend eine Kanüle (12) mit einem proximalen und einem distalen Abschnitt (20), die vorzugsweise zueinander einen rechten oder im Wesentlichen einen rechten Winkel einschließen, eine von dem proximalen Abschnitt ausgehende Handhabe (14) mit zumindest einem flächigen ersten Abschnitt (36, 38), der von einem von dem proximalen Abschnitt der Kanüle ausgehenden Mittelabschnitt (30) ausgeht oder Abschnitt von diesem ist, sowie eine Fixierungshilfe (60) mit einer Klebefläche (62) zum Fixieren der Kanülenanordnung auf einer Unterlage wie Haut eines Patienten, und
dass sich entlang des flächigen Abschnitts (36, 38) der Handhabe (14) und seitlich über diesen überstehend die Fixierungshilfe (60) mit der Klebefläche (62) erstreckt, und dass die Klebefläche der Fixierungshilfe auf der dem flächigen Abschnitt zugewandten Seite verläuf, **dadurch gekennzeichnet, dass** die Fixierungshilfe in dem den Mittelabschnitt (30) abdeckenden Bereich eine Klebefläche nicht aufweist.

2. Kanülenanordnung nach Anspruch 1,
**dadurch gekennzeichnet,**
**dass** die Fixierungshilfe wie Pflaster (60) lösbar mit der Handhabe (16) verbunden und mittels dieser fixierbar ist.

3. Kanülenanordnung nach Anspruch 1 oder 2,
**dadurch gekennzeichnet,**
**dass** die Fixierungshilfe wie das Pflaster (60) mechanisch auf dem Mittelabschnitt (30) fixiert ist.

4. Kanülenanordnung nach zumindest einem der vorhergehenden Ansprüche,
wobei der flächige Abschnitt der Handhabe durch zu dem Mittelabschnitt (30) verschwenkbare Flügel (36, 38) ausgebildet ist und an dem Mittelabschnitt eine nach Gebrauch der Kanüle (10) deren distalen Abschnitt (20) abdeckende Schutzeinrichtung (16) angelenkt ist, die um eine senkrecht zum proximalen Kanülenabschnitt verlaufende Achse (40) schwenkbar ist und im Anlieferungszustand der Kanüle auf dem Mittelabschnitt fixiert ist,
**dadurch gekennzeichnet,**
**dass** die Fixierungshilfe wie das Pflaster (60) zwischen dem Mittelabschnitt (30) und der Schutzeinrichtung (16) fixiert ist.

5. Kanülenanordnung nach zumindest einem der vorhergehenden Ansprüche,
**dadurch gekennzeichnet,**
**dass** der sich entlang jeden Flügels (36, 38) erstreckende Abschnitt der Fixierungshilfe wie das Pflaster (60) in Draufsicht eine trapezartige Geometrie mit im Bereich des Mittelabschnitts (30) kürzerer Basis aufweist.

6. Kanülenanordnung nach zumindest einem der vorhergehenden Ansprüche, wobei die Schutzeinrichtung (16) einen kanalförmigen Abschnitt mit Bodenwandung (46) zur Aufnahme des distalen Abschnitts (20) der Kanüle (10) aufweist,
**dadurch gekennzeichnet,**
**dass** die Bodenwandung (46) des kanalförmigen Abschnitts der Schutzeinrichtung (16) ein erstes Rastmittel (58) aufweist, dem ein in dem Mittelabschnitt (30) der Handhabe vorhandenes zweites Rastmittel (56) zum Verrasten der Schutzeinrichtung zugeordnet ist.

7. Kanülenanordnung nach Anspruch 6,
**dadurch gekennzeichnet,**
**dass** das erste Rastmittel (58) eine Aussparung und das zweite Rastmittel (56) ein Vorsprung ist oder umgekehrt.

8. Kanülenanordnung nach Anspruch 6 oder 7,
**dadurch gekennzeichnet,**
**dass** die Achse (40), um die die Schutzeinrichtung (16) schwenkbar ist, zwischen dem Mittelabschnitt (30) der Handhabe (14) und einer proximalen flächigen Erweiterung (52) der Schutzeinrichtung verläuft und dass die Erweiterung in die Bodenwandung (46) übergeht und quer zu der Kanüle (10) eine größere Erstreckung als die Bodenwandung aufweist.

9. Kanülenanordnung nach zumindest einem der vorhergehenden Ansprüche, deren distaler Kanülenabschnitt (20) senkrecht oder im Wesentlichen senkrecht zum proximalen Kanülenabschnitt verläuft,
**dadurch gekennzeichnet,**
**dass** bei auf dem Mittelabschnitt (30) fixierter Schutzeinrichtung (16) deren Bodenwandung (46) und die flächige Erweiterung (52) entlang Oberseite des Mittelabschnitts verläuft.

10. Kanülenanordnung nach Anspruch 8 oder 9,
**dadurch gekennzeichnet,**
**dass** bei entlang der Außenfläche des Mittelabschnitts (30) verlaufender flächiger Erweiterung (52) diese von der Längsachse des distalen Kanülenabschnitts (20) durchsetzt ist.

## Claims

1. Cannula arrangement (10) comprising a cannula (12) with a proximal and a distal section (20), which are preferably perpendicular or substantially perpendicular to one another, a handle (14) extending from the proximal section with at least one wide-area first section (36, 38) that emanates from a central section (30) which stems from the proximal section of the cannula, or is a section thereof, as well as a fixing aid (60) with an adhesive surface (62) to fix the cannula arrangement to a surface, such as the skin of a patient, and wherein the fixing aid (60) with the adhesive surface (62) extends along the wide-area section (36, 38) of the handle (14) and to the side projecting over this, and wherein the adhesive surface of the fixing aid extends on the side facing the wide-area section,
**characterized in that**
the fixing aid does not have an adhesive surface in the region covering the central section (30).

2. Cannula arrangement according to claim 1,
**characterized in that**
the fixing aid such as a plaster (60) is removably connected to the handle (16) and can be fixed through this.

3. Cannula arrangement according to claim 1 or 2,
**characterized in that**
the fixing aid such as the plaster (60) is mechanically fixed to the central section (30).

4. Cannula arrangement according to at least one of the above claims, wherein the wide-area section of the handle is formed through wings (36, 38) that can be swivelled relative to the central section (30) and a protective device (16) is articulated on the central section and covers the distal section (20) after use of the cannula (10), said device (16) can be swivelled about an axis (40) that extends perpendicularly to the proximal cannula section and in the delivery state of the cannula is fixed on the central section,
**characterized in that**
the fixing aid such as the plaster (60) is fixed between the central section (30) and the protective device (16).

5. Cannula arrangement according to at least one of the above claims,
**characterized in that**
the section of the fixing aid such as the plaster (60) extending along each wing (36, 38) in a top view has a trapezoidal geometry with a shorter base in the region of the central section (30).

6. Cannula arrangement according to at least one of the above claims, wherein the protective device (16) has a channel-shaped section with a base wall (46) to accommodate the distal section (20) of the cannula (10),
**characterized in that**
the base wall (46) of the channel-shaped section of the protective device (16) has a first catch means (58) to which is assigned a second catch means (56) provided in the central section (30) of the handle to lock the protective device in place.

7. Cannula arrangement according to claim 6,
**characterized in that**
the first catch means (58) is a recess and the second catch means (56) is a projection, or vice versa.

8. Cannula arrangement according to claim 6 or 7,
**characterized in that**
the axis (40) about which the protective device (16) can be swivelled, extends between the central section (30) of the handle (14) and a proximal wide-area extension (52) of the protective device and that the extension merges into the base wall (46) and transversely to the cannula (10) has a greater extension than the base wall.

9. Cannula arrangement according to at least one of the above claims, wherein its distal cannula section (20) extends perpendicularly or substantially perpendicularly to the proximal cannula section
**characterized in that**
if the protective device (16) is fixed on the central section (30), its base wall (46) and the wide-area extension (52) extend along the upper side of the central section.

10. Cannula arrangement according to claim 8 or 9,
**characterized in that**
if the wide-area extension (52) extends along the outer surface of the central section (30), this is intersected by the longitudinal axis of the distal cannula section (20).

## Revendications

1. Système de canule (10) comprenant une canule (12) avec une partie proximale et une partie distale (20) qui de préférence inscrivent entre elles un angle droit ou quasiment droit, une manette (14) partant de la partie proximale avec au moins une première partie en nappe (36, 38) qui part ou fait partie d'une partie médiane (30) partant de la partie proximale de la canule, ainsi qu'un moyen de fixation (60) avec une surface adhésive (62) pour fixer le système de canule sur un support tel que la peau d'un patient, sachant que le moyen de fixation (60) avec surface adhésive (62) s'étend le long de la partie en nappe (36, 38) de la manette (14) en la dépassant latéralement, et que la surface adhésive du moyen de fixation s'étend du côté orienté vers la partie en nappe,
**caractérisé en ce**
**que** le moyen de fixation ne présente pas une surface adhésive dans la zone recouvrant la partie médiane (30).

2. Système de canule selon la revendication 1,
**caractérisé en ce**
**que** le moyen de fixation tel qu'un sparadrap (60) est relié à la manette (16) de manière détachable et peut être fixé au moyen de cette dernière.

3. Système de canule selon la revendication 1 ou 2,
**caractérisé en ce**
**que** le moyen de fixation tel que le sparadrap (60) est fixé mécaniquement sur la partie médiane (30).

4. Système de canule selon au moins une des revendications précédentes, sachant que la partie en nappe de la manette est formée par des ailettes (36, 38) pivotantes par rapport à la partie médiane (30), et que sur la partie médiane est fixé de manière articulée un dispositif protecteur (16) recouvrant sa partie distale (20) après usage de la canule (10), lequel peut pivoter autour d'un axe (40) perpendiculaire à la partie proximale de la canule et est fixé sur la partie médiane dans l'état de livraison de la canule,
**caractérisé en ce**
**que** le moyen de fixation tel que le sparadrap (60) est fixé entre la partie médiane (30) et le dispositif protecteur (16).

5. Système de canule selon au moins une des revendications précédentes,
**caractérisé en ce**
**que** vue en élévation, la partie du moyen de fixation tel que le sparadrap (60) s'étendant le long de chaque ailette (36, 38) présente une géométrie trapézoïdale avec petite base dans la zone de la partie médiane (30).

6. Système de canule selon au moins une des revendications précédentes, dans lequel le dispositif protecteur (16) présente une partie en forme de conduit avec paroi de fond (46) destinée à loger la partie distale (20) de la canule (10),
**caractérisé en ce**
**que** la paroi de fond (46) de la partie en forme de conduit du dispositif protecteur (16) présente un premier moyen de crantage (58) auquel est associé un second moyen de crantage (56) présent dans la partie médiane (30) de la manette afin d'enclencher le dispositif protecteur.

7. Système de canule selon la revendication 6,
**caractérisé en ce**
**que** le premier moyen de crantage (58) est un évidement et le second moyen de crantage (56) est un partie saillante, ou inversement.

8. Système de canule selon la revendication 6 ou 7,
**caractérisé en ce**
**que** l'axe (40), autour duquel le dispositif protecteur (16) peut pivoter, s'étend entre la partie médiane (30) de la manette (14) et un élargissement proximal en nappe (52) du dispositif protecteur, et que l'élargissement se prolonge dans la paroi de fond (46) et présente transversalement à la canule (10) une plus grande extension que la paroi de fond.

9. Système de canule selon au moins une des revendications précédentes, dont la partie distale (20) de la canule est perpendiculaire ou quasiment perpendiculaire à la partie proximale de la canule,
**caractérisé en ce**
**que** lorsque le dispositif protecteur (16) est fixé sur la partie médiane (30), sa paroi de fond (46) et l'élargissement en nappe (52) s'étendent le long de la face supérieure de la partie médiane.

10. Système de canule selon la revendication 8 ou 9,
**caractérisé en ce**
**que** lorsque l'élargissement en nappe (52) s'étend le long de la face extérieure de la partie médiane (30), il est traversé par l'axe longitudinal de la partie distale (20) de la canule.
